# EUROPÄISCHE PATENTANMELDUNG

(11) **EP 4 191 173 A1**
(43) Veröffentlichungstag der Anmeldung: **07.06.2023**
(21) Anmeldenummer: 22203602.2
(22) Anmeldetag: 25.10.2022
(51) Int. Cl.: F25D 23/06, F25D 13/02

(54) **TRENNWAND**

(30) Priorität: 03.12.2021 DE 102021131981
(71) Anmelder: Binder GmbH, 78532 Tuttlingen (DE)
(72) Erfinder: Straub, Gerhard, 78166 Donaueschingen (DE); Arabul, Ahmet, 78532 Tuttlingen (DE); Leidolt, Richard, 78224 Singen (DE)
(74) Vertreter: Westphal, Mussgnug & Partner Patentanwälte mbB

(57) **Zusammenfassung**

Trennwand (10, 10') zum lösbaren Einsetzen in einen Laborschrank (100), beispielsweise einen Inkubator, Kälteschrank, Wärmeschrank, Trockenschrank oder Klimaschrank, wobei die Trennwand (10, 10') einen im Wesentlichen rechteckigen, flachen Grundkörper (12) mit einer Unterkante (12a), einer Oberkante (12b), einer Vorderkante (12c), einer Rückkante (12d) sowie zwei parallelen Seitenflächen (12e, 12f) aufweist, wobei an dem Grundkörper (12) wenigstens ein zur Vorderkante (12c) offener Einsetzhaken (20) und wenigstens ein Federelement (30) zur Erzeugung einer Kraft in Richtung zur Vorderkante (12c) hin angeordnet sind.

## Beschreibung

Die Erfindung betrifft eine Trennwand zum lösbaren Einsetzen in einen Laborschrank, beispielsweise einen Inkubator, Kälteschrank, Wärmeschrank, Trockenschrank oder Klimaschrank.

Bekannt sind Laborschränke mit einem wenigstens eine Außentür aufweisenden Gehäuse, welches wenigstens einen Innenraum aufweist. Oft weisen derartige Laborschränke Vorrichtungen auf, mit welchen im Innenraum eine definierte Temperatur und/oder definierte Klimabedingungen, beispielsweise eine definierte Feuchte oder Gaskonzentration, einstellbar sind. Beispielsweise sind Inkubatoren, Kälteschränke, Wärmeschränke, Trockenschränke oder Klimaschränke bekannt. Um zu verhindern, dass beim Öffnen der Außentür die klimatischen Bedingungen im Innenraum gestört werden, ist es bekannt, den Innenraum durch eine zusätzliche Innentür zu verschließen. Die Außentüre dient im Wesentlichen der Dämmung zwischen den Innenraum des Laborschranks und der Umgebung. Die Innentür verhindert den Luftaustausch des Innenraums mit der Umgebung beim Öffnen der Außentüre. Die Innentür kann aus transparentem Material gefertigt sein, was es ermöglicht, bei geöffneter Außentür einen Blick in den Innenraum zu werden, ohne die klimatischen Bedingungen im Innenraum zu stören. Dabei besteht auch die Möglichkeit, die Innentür in mehrere kleinere Türen aufzuteilen, die einen Zugriff lediglich in einen Teilbereich des Innenraums erlauben. Einen Inkubator mit einer derartigen Innentür zeigt beispielsweise die DE 10 2004 049 210 A1. Ein Fach erstreckt sich dabei über die gesamte Breite des Laborschranks.

Die US 2005/0244306 A1 offenbart einen Klimaschrank, bei welchem der Innenraum durch Boxen in mehrere Teilräume aufgeteilt ist und mehrere Innentüren die kleineren Teilräume verschließen, so dass das Klima in den verbleibenden Teilräumen möglichst wenig gestört wird, wenn in einen der Teilräume Probengut eingelagert wird oder aus dem Teilraum Probengut entnommen wird. Eine derartige Ausgestaltung ist wenig flexibel und sehr aufwändig.

Die Aufgabe der Erfindung besteht daher darin, eine Möglichkeit bereitzustellen, den Innenraum eines Laborschranks auf einfache Art und Weise flexibel in kleinere Bereiche einteilen zu können.

Die Aufgabe wird erfindungsgemäß gelöst durch eine Trennwand zum lösbaren Einsetzen in einen Laborschrank mit den Merkmalen des Patentanspruchs 1.

Vorteilhafte Ausgestaltungen und Weiterbildungen der Erfindung sind in den abhängigen Ansprüchen angegeben.

Die erfindungsgemäße Trennwand zum lösbaren Einsetzen in einen Laborschrank, beispielsweise einen Inkubator, Kälteschrank, Wärmeschrank, Trockenschrank oder Klimaschrank, weist einen im Wesentlichen rechteckigen, flachen Grundkörper mit einer Unterkante, einer Oberkante, einer Vorderkante, einer Rückkante sowie zwei parallelen Seitenflächen auf, wobei an dem Grundkörper wenigstens ein zur Vorderkante offener Einsetzhaken und wenigstens ein Federelement zur Erzeugung einer Kraft in Richtung zur Vorderkante hin angeordnet sind. Eine derartige Ausgestaltung ermöglicht ein einfaches Einsetzen und Lösen der Trennwand ohne zusätzliche Werkzeuge, da durch das Federelement eine Fixierung erfolgen kann, welche auf einfache Art auch wieder gelöst werden kann. Zudem ermöglicht eine derartige Trennwand eine flexible Kompartimentierung: Je nach Anforderung kann die Trennwand eingesetzt werden, um kleinere Teilbereiche zu erhalten, oder entfernt werden, falls größere Teilbereiche erforderlich sind.

Die erfindungsgemäße Trennwand kann insbesondere an einem insbesondere horizontal angeordneten Boden oder Zwischenboden eines Laborschranks derart angeordnet werden, dass sie senkrecht zu dem Boden oder Zwischenboden und damit insbesondere vertikal ausgerichtet ist. Zusätzlich zu einer grundsätzlich bekannten Einteilung in Fächer, welche durch die Zwischenböden erreicht wird, kann mit der erfindungsgemäßen Trennwand eine weitere Einteilung auf einer Fachebene in kleinere Fächer erreicht werden, um damit die Gefahr der Vertauschung von Proben, insbesondere wenn auf einer Fachebene mehrere Türen nebeneinander angeordnet sind, zu verringern.

Vorzugsweise sind an dem Grundkörper wenigstens zwei Einsetzhaken angeordnet, wodurch die Stabilität der Fixierung erhöht werden kann.

Besonders bevorzugt ist der Einsetzhaken an der Unterkante angeordnet, was eine besonders platzsparende Anordnung ermöglicht.

Gemäß einer besonders bevorzugten Weiterbildung der Erfindung ist das Federelement durch eine abgekantete Lasche, insbesondere eine zweifach abgekantete Lasche, gebildet. Ein derartiges Federelement kann auf einfache Art und Weise durch Biegen hergestellt werden.

Vorteilhafterweise ist das Federelement in etwa V-förmig ausgebildet, wodurch sich eine platzsparende Ausgestaltung des Federelements ergibt. Ein derartiges Federelement kann zudem besonders einfach zu handhaben sein.

Besonders bevorzugt ist ein freies Ende des Federelements als Auflageelement ausgebildet, was die Handhabung beim Einsetzen der Trennwand vereinfachen kann.

Eine vorteilhafte Weiterbildung der Erfindung sieht vor, dass an der Unterkante wenigstens ein Stützelement, welches im Wesentlichen senkrecht zu einer Seitenfläche ausgebildet ist, angeordnet ist, wobei vorzugsweise wenigstens zwei Stützelemente, welche insbesondere in unterschiedliche Richtungen weisen, an der Unterkante angeordnet sind. Ein derartiges Stützelement kann die Standfestigkeit der Trennwand erhöhen und die Gefahr eines Verkippens der Trennwand in die Richtung, in welche das Stützelement weist, verringern.

Ist das Federelement gemäß einer bevorzugten Ausführungsform an der Unterkante angeordnet, kann sich eine platzsparende Ausgestaltung ergeben. In einer alternativen besonders bevorzugten Ausführungsform kann das Federelement an der Rückkante in räumlicher Nähe zur Unterkante oder an dem Stützelement angeordnet sein, was eine einteilige Ausbildung zwischen Federelement und Grundkörper und insbesondere eine Fertigung durch einen Stanz-Biege-Prozess ermöglichen kann.

Vorzugsweise weist der Grundkörper eine Vielzahl von Löchern auf und ist insbesondere als Lochblech ausgebildet. Durch eine derartige Ausgestaltung der Trennwand kann verhindert werden, dass die Trennwand die räumliche Temperatur- oder Feuchteverteilung im Innenraum des Laborschranks beeinträchtigt.

Vorteilhafterweise ist die Trennwand aus Metall gefertigt. Eine Trennwand aus Metall ermöglicht eine einfache Reinigung und insbesondere Sterilisation.

Gemäß einer besonders bevorzugten Ausführungsform der Erfindung ist die Trennwand als Stanz-Biege-Element und insbesondere einteilig ausgebildet. Eine derartige Trennwand ermöglicht eine besonders einfache und kostengünstige Herstellung.

Ein erfindungsgemäßer Laborschrank, beispielsweise Inkubator, Kälteschrank, Wärmeschrank, Trockenschrank oder Klimaschrank, mit einem wenigstens eine Außentür aufweisenden Gehäuse, welches wenigstens einen Innenraum aufweist, der durch eine Innentür verschließbar ist, wobei der Innenraum eine Bodenwand, eine Deckwand, eine Rückwand und zwei Seitenwände aufweist, wobei zwischen den beiden Seitenwänden parallel zur Bodenwand wenigstens ein Zwischenboden angeordnet ist, wobei entweder die Bodenwand und/oder der Zwischenboden eine erste Ausnehmung und eine Anschlagkante aufweisen, weist eine erfindungsgemäße Trennwand auf, welche an der Bodenwand und/oder dem Zwischenboden derart angeordnet ist, dass der Einsetzhaken in die Ausnehmung eingreift und das Federelement an der Anschlagkante anliegt.

Ausführungsformen der Erfindung werden anhand der nachfolgenden Figuren ausführlich erläutert. Es zeigen
- Figur 1: eine perspektivische Ansicht auf ein erstes Ausführungsbeispiel einer Anordnung von in einem Gehäuse eines Laborschranks angeordneten Komponenten, insbesondere umfassend zwei Zwischenböden sowie zwei an den Zwischenböden angeordneten Trennwänden, wobei das Gehäuse ausgeblendet ist,
- Figur 2: eine Ansicht von oben auf die Anordnung gemäß Figur 1,
- Figur 3: eine Ausschnittvergrößerung des Details Z aus Figur 2, welche ein Federelement zeigt,
- Figur 4: eine Ansicht von vorne auf die Anordnung gemäß Figur 1,
- Figur 5: eine Ansicht von schräg unten auf die Anordnung gemäß Figur 1,
- Figur 6: eine Ausschnittvergrößerung des Details Y aus Figur 5, welche einen Einsetzhaken zeigt,
- Figur 7: eine Ausschnittvergrößerung einer weiteren perspektivischen Ansicht der Anordnung gemäß Figur 1,
- Figur 8: eine perspektivische Ansicht auf ein zweites Ausführungsbeispiel einer Anordnung von in einem Gehäuse eines Laborschranks angeordneten Komponenten, insbesondere umfassend zwei Zwischenböden sowie zwei an den Zwischenböden angeordneten Trennwänden, wobei das Gehäuse ausgeblendet ist,
- Figur 9: eine Ansicht von oben auf die Anordnung gemäß Figur 8,
- Figur 10: eine Ansicht von vorne auf die Anordnung gemäß Figur 8,
- Figur 11: eine Ansicht von schräg unten auf die Anordnung gemäß Figur 8,
- Figur 12: eine teilweise geschnittene weitere perspektivische Ansicht der Anordnung gemäß Figur 8,
- Figur 13: eine Ausschnittvergrößerung des Details A aus Figur 11, welche einen Einsetzhaken zeigt,
- Figur 14: eine Ausschnittvergrößerung des Details B aus Figur 11, welche ein Federelement zeigt,
- Figur 15: das Federelement gemäß Figur 14 in einer weiteren perspektivischen Ansicht,
- Figur 16: das Federelement gemäß Figur 14 in einer weiteren perspektivischen Ansicht vor dem Einsetzen der Trennwand,
- Figur 17: das Federelement gemäß Figur 14 in einer weiteren perspektivischen Ansicht mit der Trennwand in einem eingesetzten Zustand,
- Figur 18: eine Seitenansicht der Trennwand der Anordnung gemäß Figur 8,
- Figur 19: eine Ausschnittvergrößerung einer perspektivischen Ansicht der Anordnung gemäß Figur 18.
- Figur 20: eine perspektivische Ansicht eines Ausführungsbeispiels eines Laborschranks mit darin angeordneten Komponenten gemäß Figur 8,
- Figur 21: eine weitere perspektivische Ansicht des Laborschranks gemäß Figur 20 mit teilweise ausgezogenen Zwischenböden,
- Figur 22: eine Frontansicht des Laborschranks gemäß Figur 20,
- Figur 23: eine Schnittansicht des Laborschranks gemäß Figur 20 und
- Figur 24: eine weitere perspektivische Ansicht des Laborschranks gemäß Figur 20.

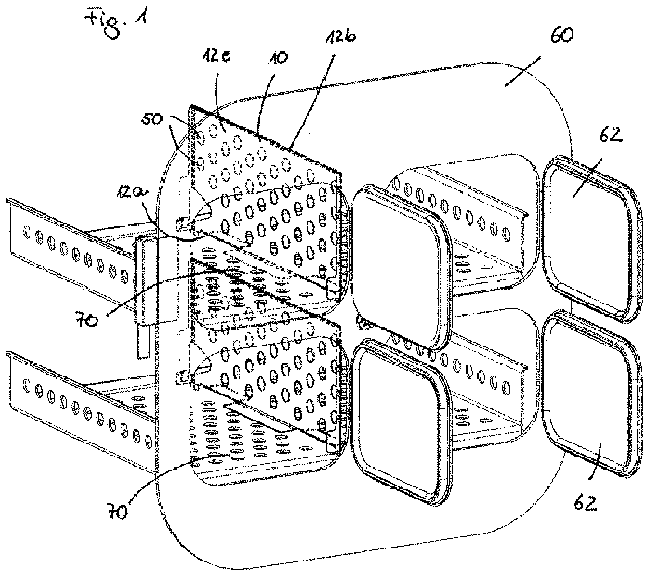

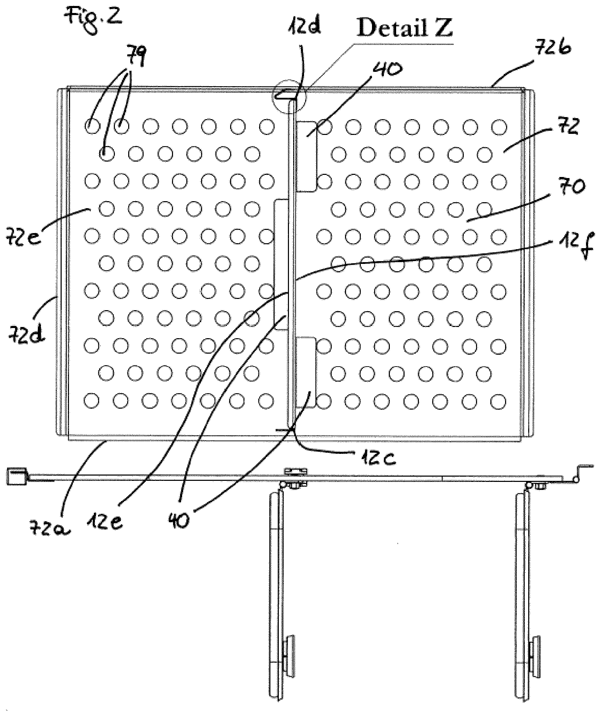

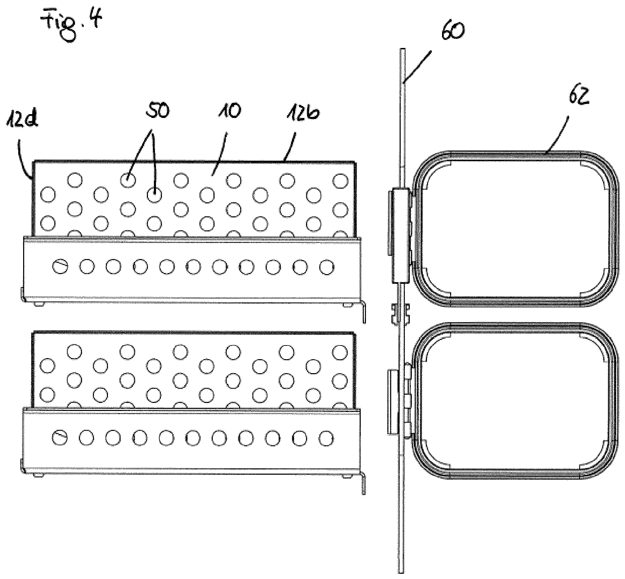

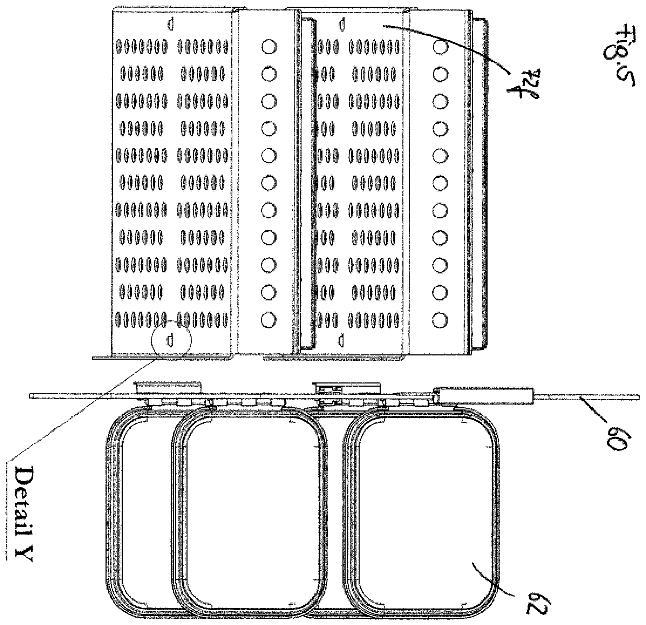

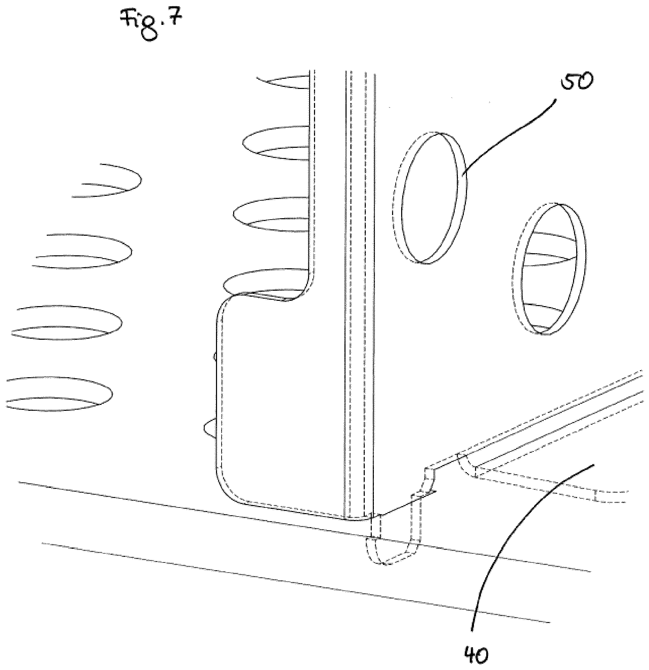

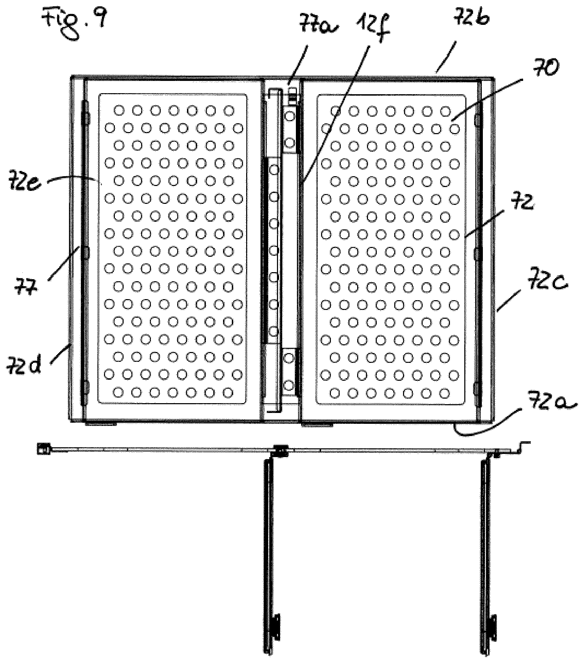

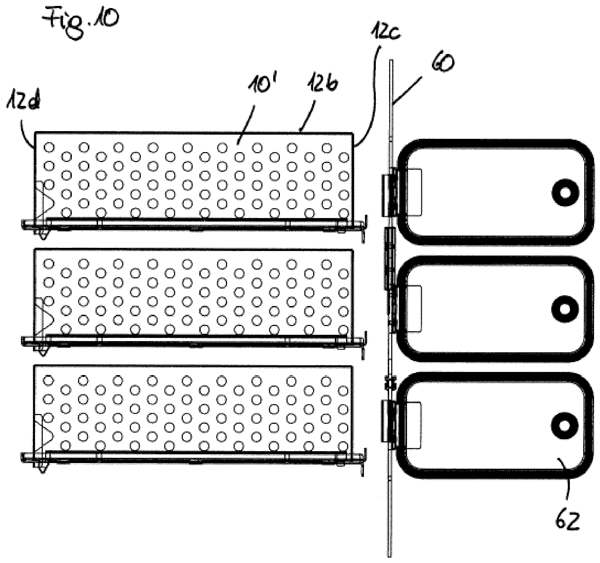

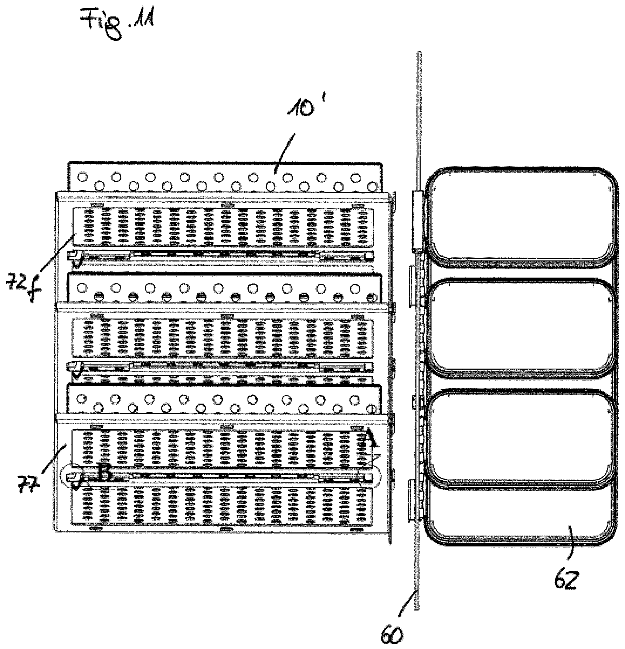

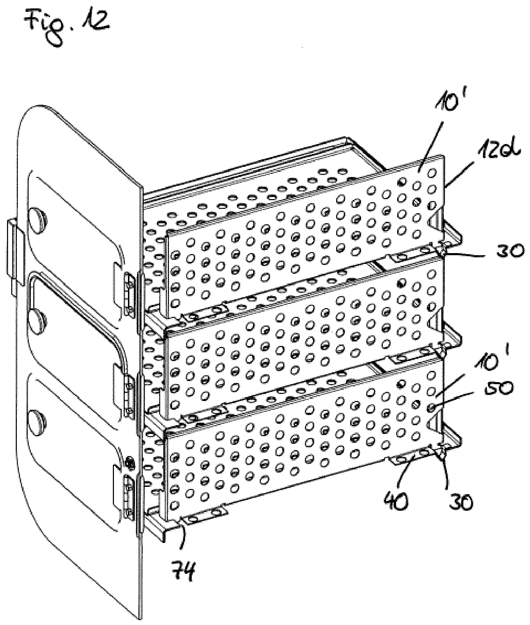

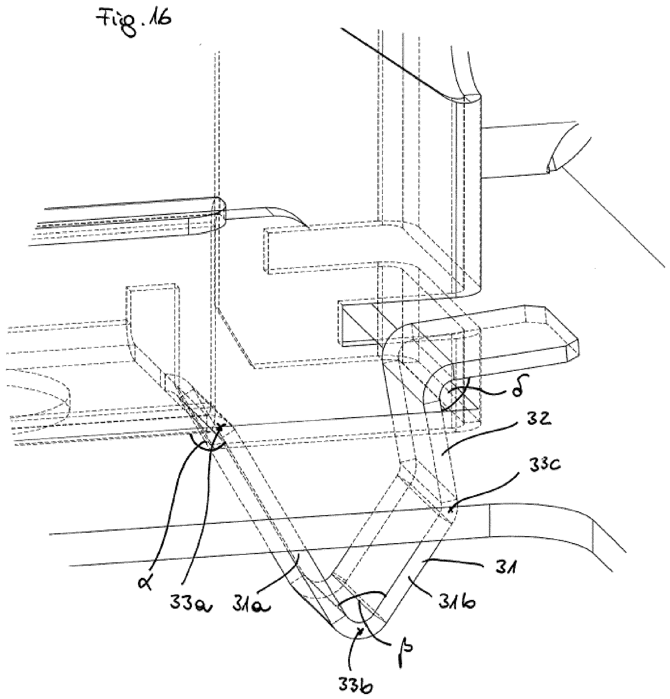

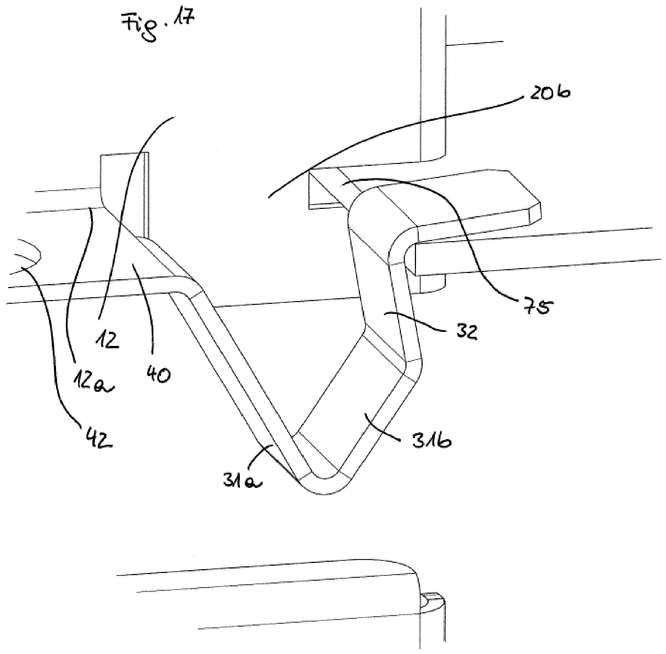

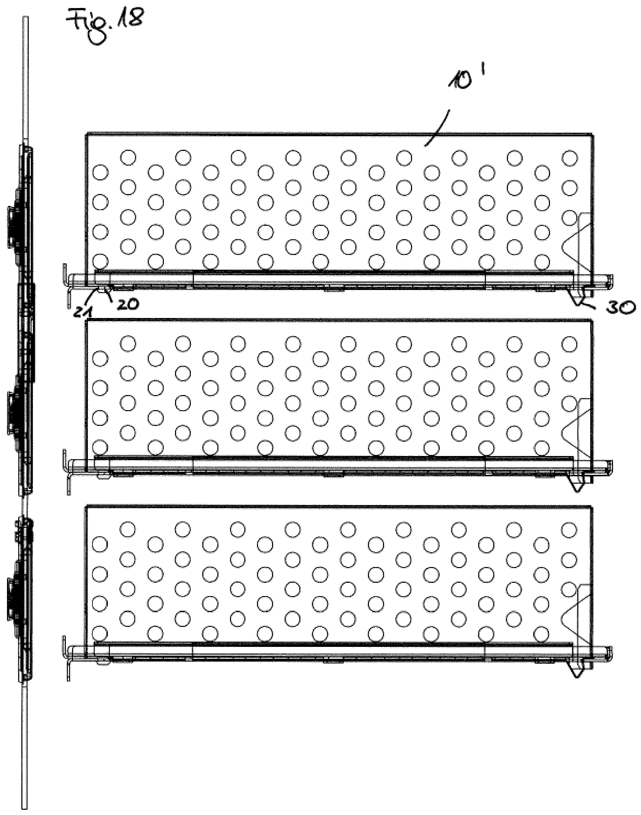

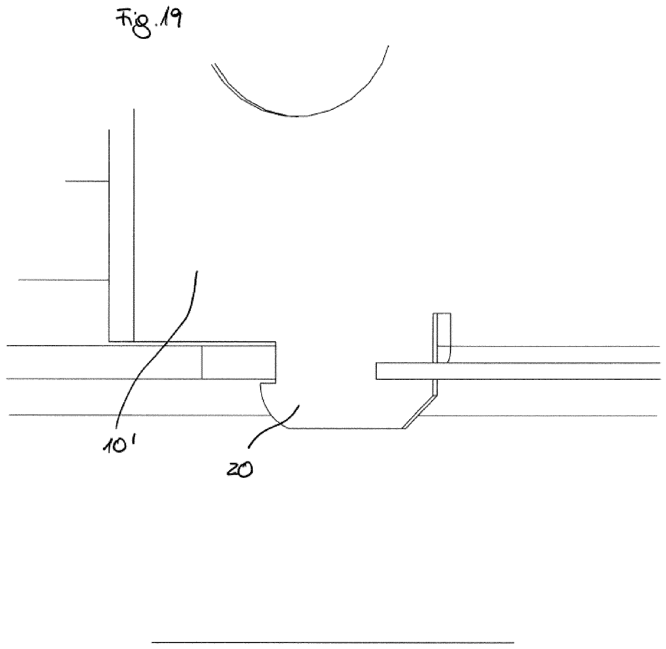

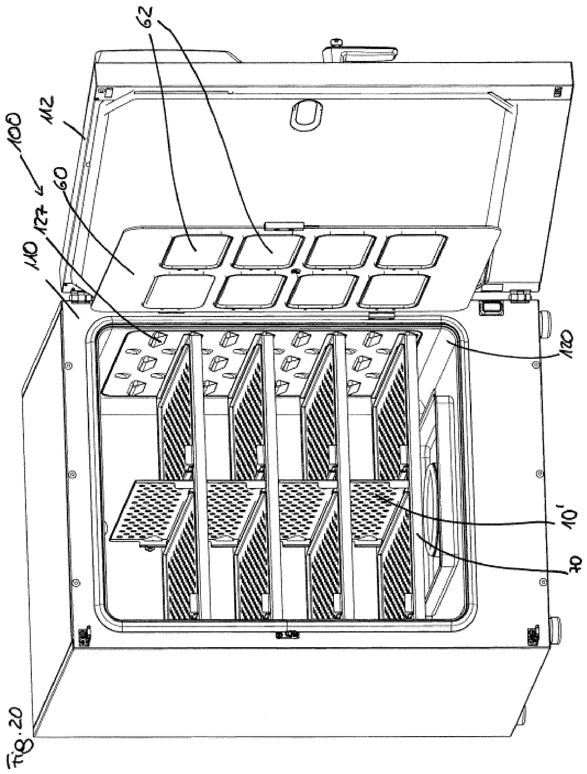

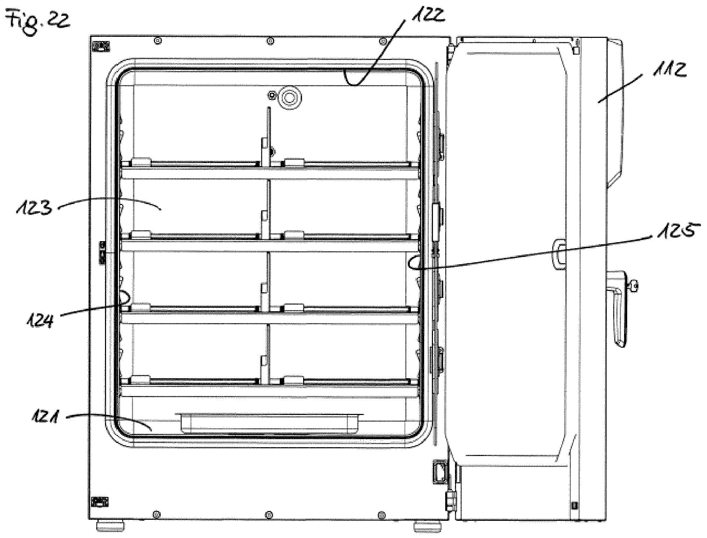

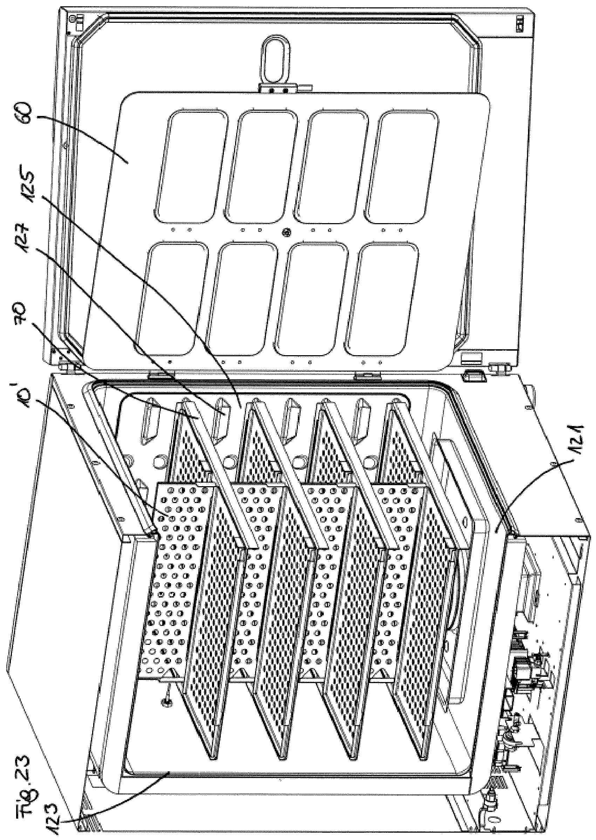

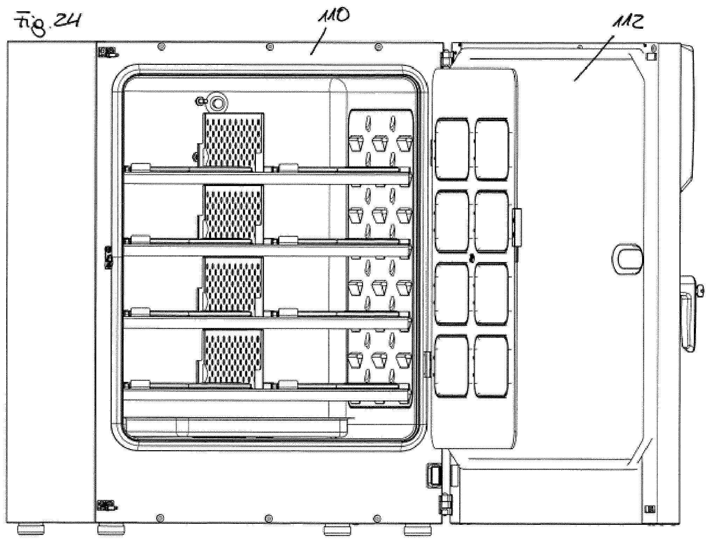

Die Figuren 1 bis 8 zeigen verschiedene Ansichten eines ersten Ausführungsbeispiels einer Anordnung von in einem Gehäuse eines Laborschranks angeordneten Komponenten, wobei das Gehäuse ausgeblendet ist, während Figuren 9 bis 19 verschiedene Ansichten eines zweiten Ausführungsbeispiels einer Anordnung von in einem Gehäuse eines Laborschranks angeordneten Komponenten, wobei das Gehäuse ausgeblendet ist, zeigt. Die Figuren 20 bis 24 zeigen verschiedene Ansichten eines Ausführungsbeispiels eines Laborschranks 100, in welchem die Anordnung von Komponenten gemäß dem in den Figuren 9 bis 19 dargestellten zweiten Ausführungsbeispiel angeordnet ist. Gleiche oder funktionsgleiche Teile werden mit gleichen Bezugsziffern bezeichnet, wobei zur besseren Übersicht nicht sämtliche Bezugsziffern in sämtlichen Figuren dargestellt sind. Die nachfolgende Beschreibung bezieht sich soweit nicht explizit anders angegeben auf beide Ausführungsbeispiele von Anordnungen. Insbesondere können beide Ausführungsbeispiele der Anordnungen in dem Laborschrank 100 gemäß der Figuren 20 bis 24 angeordnet sein.

Figur 1 zeigt unter anderem eine Trennwand 10 zum lösbaren Einsetzen in einen in dieser Figur nicht dargestellten Laborschrank 100, beispielsweise einen Inkubator, Kälteschrank, Wärmeschrank, Trockenschrank oder Klimaschrank. Die Trennwand 10 weist einen im Wesentlichen rechteckigen, flachen Grundkörper 12 mit einer Unterkante 12a, einer Oberkante 12b, einer Vorderkante 12c, einer Rückkante 12d sowie zwei parallelen Seitenflächen 12e, 12f auf, wobei die Unterkante 12a insbesondere parallel zur Oberkante 12b und die Vorderkante 12c insbesondere parallel zur Rückkante 12d angeordnet sind. Der Abstand zwischen den beiden Seitenflächen 12e, 12f ist dabei insbesondere wesentlich geringer als die Länge der Vorderkante 12c und die Länge der Unterkante 12a, so dass sich ein flacher Grundkörper 12 ergibt. Der Grundkörper 12 kann insbesondere Löcher 50 aufweisen und beispielsweise als Lochblech ausgebildet sein, um die Luftzirkulation im Innenraum des Laborschranks 100 möglichst wenig zu beeinträchtigen.

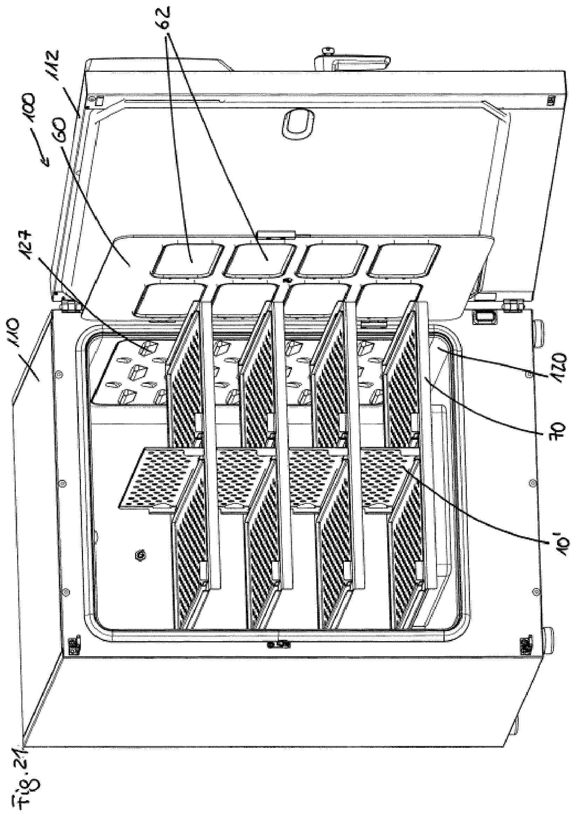

Wie in den Figuren 20 bis 24 erkennbar, weist der Laborschrank 100 üblicherweise ein wenigstens eine Außentür 112 aufweisenden Gehäuse 110 auf, welches wenigstens einen Innenraum 120 aufweist, der durch eine Innentür 60 verschließbar ist. Die Innentür 60 kann dabei mehrere kleine Türen 62 aufweisen, welche einen Zugriff nur zu einem Teilbereich des Innenraums 120 ermöglichen. Dabei weist der Innenraum 120 üblicherweise eine Bodenwand 121, eine Deckwand 122, eine Rückwand 123 und zwei Seitenwände 124, 125 auf. Dabei sind die Seitenwände 124, 125 und die Rückwand 123 üblicherweise im Wesentlichen vertikal angeordnet, während die Bodenwand 121 und die Deckwand 122 üblicherweise horizontal angeordnet sind. Zwischen den beiden Seitenwänden 124, 125 ist parallel zur Bodenwand 121 wenigstens ein Zwischenboden 70 angeordnet, wobei in dem in Figur 1 dargestellten Ausführungsbeispiel zwei Zwischenböden 70 dargestellt sind. Der Zwischenboden 70 weist einen im Wesentlichen rechteckigen, flachen Grundkörper 72 mit einer Vorderkante 72a, einer Rückkante 72b und zwei parallelen Seitenkanten 72c, 72d auf sowie einer Oberseite 72e und einer Unterseite 72f auf. Der Abstand zwischen der Oberseite 72e und der Unterseite 72f ist dabei insbesondere wesentlich geringer als die Länge der Vorderkante 72a und die Länge der Seitenkanten 72c, 72d, so dass sich ein flacher Grundkörper 72 ergibt. Der Grundkörper 72 kann insbesondere Löcher 79 aufweisen und beispielsweise als Lochblech ausgebildet sein, um die Luftzirkulation im Innenraum 120 des Gehäuses 110 möglichst wenig zu beeinträchtigen. Der Zwischenboden 70 kann in entsprechende Aufnahmeschienen oder Sicken 127, welche im Innenraum 120 des Gehäuses 110 angeordnet sind, eingeschoben werden (vgl. insbesondere Figur 21).

Bei bestimmungsgemäßem Gebrauch der Trennwand 10 im in den Innenraum 120 des Laborschranks 100 eingesetzten Zustand und insbesondere an dem Zwischenboden 70 angeordneten Zustand weist die Unterkante 12a nach unten und die Oberkante 12b nach oben, so dass die Trennwand 10 im Wesentlichen vertikal angeordnet ist. Weiterhin ist bei bestimmungsgemäßem Gebrauch der Trennwand 10 vorzugsweise die Vorderkante 12c zur Innentür weisend angeordnet, während die Rückkante 12d vorzugsweise zur Rückwand des Innenraums gerichtet angeordnet ist. Eine Anordnung derart, dass die Vorderkante 12c zur Rückwand und die Rückkante 12d zur Innentür gerichtet angeordnet sind, ist jedoch ebenfalls möglich.

An dem Grundkörper 12 der Trennwand 10 ist wenigstens ein zur Vorderkante 12c offener Einsetzhaken 20, vorzugsweise zwei zur Vorderkante 12c offene Einsetzhaken 20, angeordnet, insbesondere an der Unterkante 12a der Trennwand. Der Einsetzhaken 20 kann eine Einführöffnung 21 aufweisen, welche zur Vorderkante 12c offen ist. Der Einsetzhaken 20 weist insbesondere dieselbe Dicke auf wie der Grundkörper 12 und kann damit insbesondere in Verlängerung des Grundkörpers 12 an der Unterkante 12a angeordnet sein. Insbesondere kann der Einsetzhaken 20 bei einem Ausstanzen der Trennwand 10 im gleichen Arbeitsschritt ausgebildet werden.

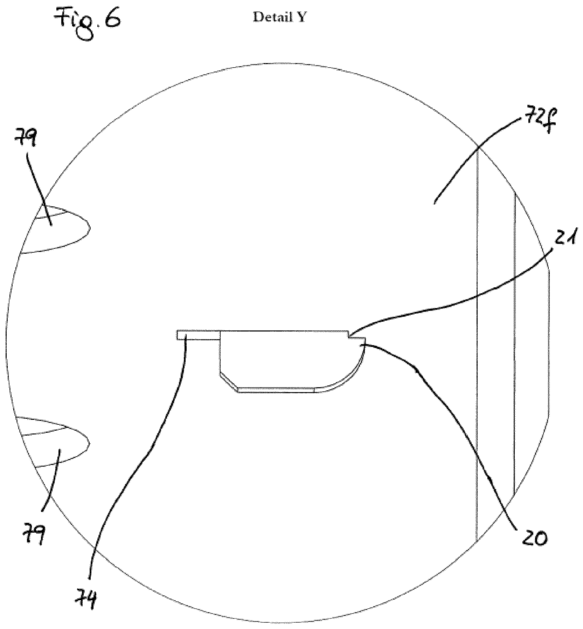

Bei einer Anordnung der Trennwand 10 an dem Zwischenboden 70 wird der Einsetzhaken 20 in eine in dem Zwischenboden 70 angeordnete Ausnehmung 74 insbesondere derart eingesetzt, dass der Rand der Ausnehmung 74 in die Einführöffnung 21 des Einsetzhakens 20 eingreift. Dabei durchgreift der Einsetzhaken 20 die Ausnehmung 74 derart, dass der Grundkörper 20 mit der Unterkante 12a an der Oberseite 72e des Zwischenbodens 70 anliegt, während das freie Ende des Einsetzhakens an der Unterseite 72f des Zwischenbodens 70 anliegt (vgl. Figur 6). Die Trennwand 10 kann in entsprechender Weise auch an dem Boden des Innenraums 120 angeordnet werden.

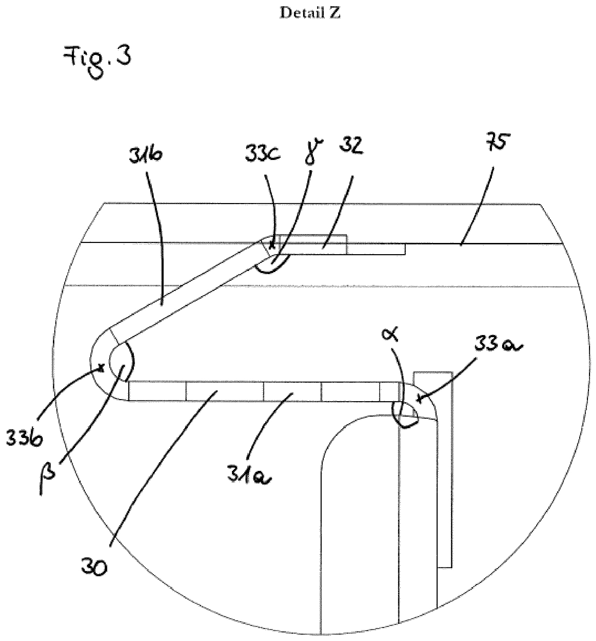

An dem Grundkörper 12 der Trennwand 10 ist wenigstens ein Federelement 30 zur Erzeugung einer Kraft 12c in Richtung zur Vorderkante 12c hin angeordnet (vgl. insbesondere Figur 3). Ein derartiges Federelement 30 bewirkt insbesondere, dass die Kante der Ausnehmung 74 gegen den Hakengrund des Einsetzhakens 20 gedrückt wird, so dass eine zuverlässige Fixierung der Trennwand 10 erreicht werden kann.

Das Federelement 30 kann beispielsweise durch eine abgekantete Lasche 31, insbesondere eine zweifach abgekantete Lasche 31, gebildet sein. Die Lasche 31 kann dabei an einer ersten Knicckante 33a gegen den Grundkörper 12 abgekantet sein, so dass ein erster Abschnitt 31a der Lasche 31 in einem Winkel α gegen den Grundkörper 12 geneigt angeordnet ist. Die Knickkante 33a beispielsweise parallel zur Rückkante 12d verlaufen. Der Winkel α kann beispielsweise etwa 90° betragen. Vorzugsweise ist die Lasche 31 zweifach abgekantet. Dazu kann die Lasche 31 an einer zweiten Knickkante 33b, welche insbesondere parallel versetzt zur ersten Knickkante 33a angeordnet ist, abgekantet sein, so dass sich ein zweiter Abschnitt 31b ergibt, welcher in einem Winkel β gegen den ersten Abschnitt 31a geneigt angeordnet ist. Der Winkel β ist vorzugsweise kleiner als 90° und kann beispielsweise zwischen 20° und 60°, vorzugsweise zwischen 30° und 40°, betragen. Insbesondere ist das Federelement 30 in etwa V-förmig ausgebildet.

Ein freies Ende des Federelements 30 kann als Auflageelement 32 ausgebildet sein und dazu nochmals abgekantet sein. Insbesondere kann das Auflageelement 32 an einer dritten Knickkante 33c gegen den zweiten Abschnitt 31b in einem Winkel γ geneigt angeordnet sein.

In dem vorliegenden Ausführungsbeispiel ist das Federelement 30 an der Rückkante 12d in räumlicher Nähe zur Unterkante 12a (vgl. Fig. 1) angeordnet. Eine derartige Anordnung ermöglicht eine einteilige Anordnung zwischen dem Federelement 30 und dem Grundkörper 12 sowie eine Ausgestaltung der Trennwand 10 als Stanz-Biege-Element.

Bei einer Anordnung der Trennwand 10 an dem Zwischenboden 70 liegt das Auflageelement 32 an einer Anschlagkante 75 an. Das Federelement 30 ist derart ausgebildet, dass bei Eingriff des Einsetzhakens 20 in die Ausnehmung 74 des Zwischenbodens und Anlage des Auflageelements 32 an der Anschlagkante 75 eine Kraft erzeugt wird, welche die Kante der Ausnehmung 74 in den Hakengrund des Einsetzhakens 20 drückt.

An der Unterkante 12a des Grundkörpers 12 kann wenigstens ein Stützelement 40, welches im Wesentlichen senkrecht zu einer der Seitenflächen 12e, 12f ausgebildet ist, angeordnet sein, wobei vorzugsweise wenigstens zwei, in dem vorliegenden Ausführungsbeispiel drei, Stützelemente 40 an der Unterkante 12a angeordnet sind. Von den drei Stützelementen 40 weist das mittlere in die gegenüberliegende Richtung als die beiden äußeren, um einen möglichst stabilen Stand der Trennwand 10 ermöglichen zu können.

Die Trennwand 10 ist insbesondere aus Metall gefertigt. Besonders bevorzugt ist die Trennwand 10 einteilig ausgebildet. Die Trennwand 10 ist insbesondere als Stanz-Biege-Element ausgebildet.

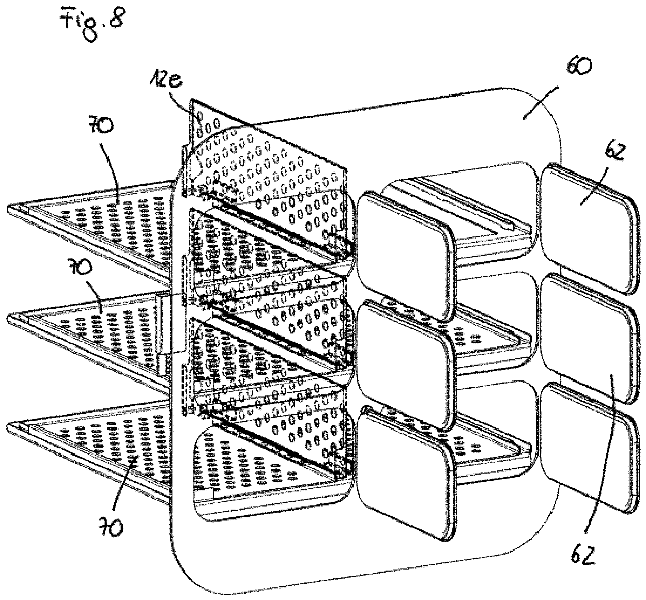

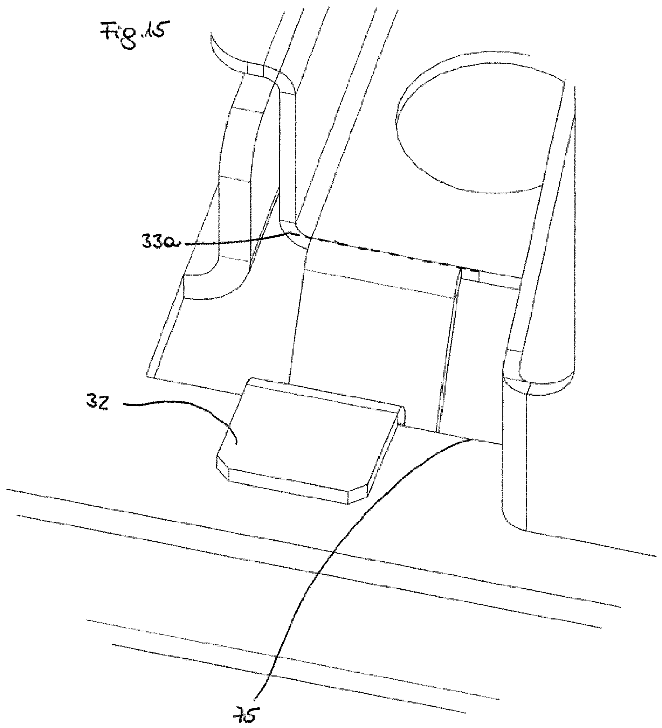

Das in den Figuren 8 bis 19 dargestellte zweite Ausführungsbeispiel unterscheidet sich im Wesentlichen durch die Anordnung des Federelements 30 an dem Grundkörper 12 der Trennwand 10'. Das Federelement 30 kann statt an der Rückkante 12d an dem Stützelement 40 angeordnet sein (vgl. insbesondere Figur 15). Die erste Knickkante 33a kann dabei insbesondere senkrecht zur Ebene der Seitenflächen 12e, 12f angeordnet sein. In diesem Ausführungsbeispiel ist das Auflageelement 32 in sich in einem Winkel δ abgewinkelt ausgebildet, um mit dem freien Ende der Lasche 31 auf der Oberseite 72e des Zwischenbodens 70 aufliegen zu können. Der Winkel δ kann beispielsweise zwischen 80° und 90° betragen.

Der Zwischenboden 70 umfasst in diesem Ausführungsbeispiel ein rahmenartiges Element 77 mit einem Mittelsteg 77a, wobei in dem Mittelsteg 77a die Ausnehmung 74 und die Anschlagkante 75 angeordnet sind, so dass die Trennwand 10' an dem Mittelsteg 77a angeordnet werden kann. Seitlich des Mittelstegs 77a kann jeweils ein Lochblech in das rahmenartige Element 77 eingelegt sein.

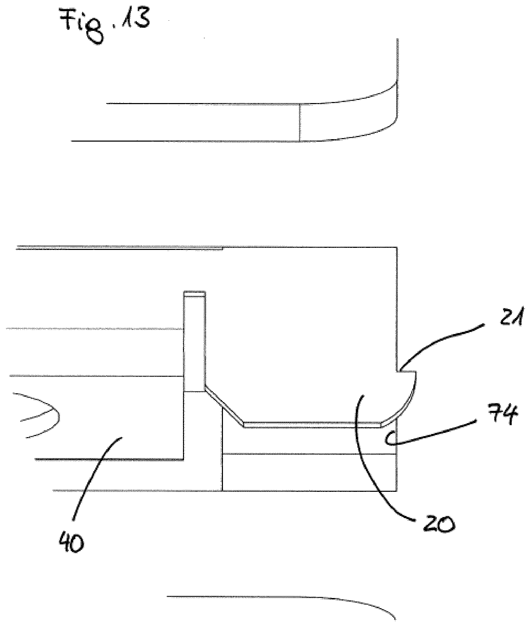

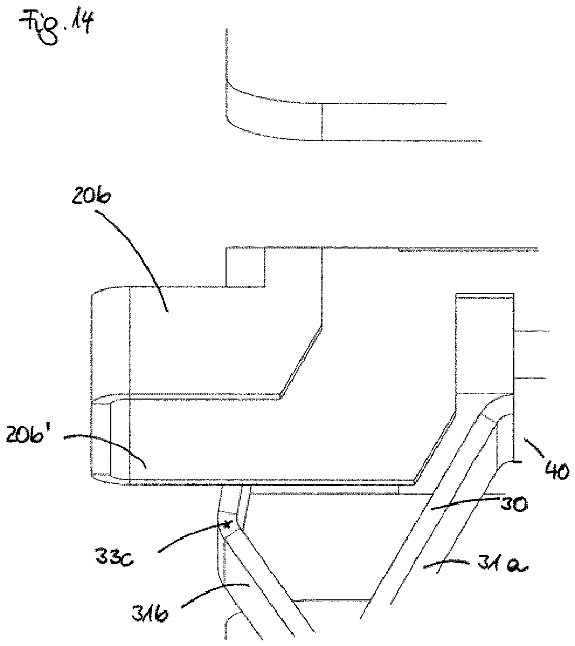

Das zweite Ausführungsbeispiel der Trennwand 10' unterscheidet sich zudem von dem ersten Ausführungsbeispiel der Trennwand 10 dadurch, dass nur der in räumlicher Nähe zur Vorderkante 12c angeordnete Einsetzhaken 20 zur Vorderkante 12c hin offen ausgebildet ist (vgl. Figur 13), während ein in räumlicher Nähe zur Rückkante 12d angeordneter Einsetzhaken 20b zur Rückkante 12d hin offen ausgebildet ist (vgl. Figur 14). Der rückwärtige Einsetzhaken 20b dient damit zur Stabilisation. Es erfolgt jedoch kein Drücken des Rands der Ausnehmung 74 zum Hakengrund des Einsetzhakens 20b durch das Federelement 30.

Weiterhin können aufgrund einer U-förmigen Umbiegung der Rücckante 12d zwei parallel versetzt zueinander angeordnete Einsetzhaken 20b, 20b' ausgebildet sein (vgl. Figuren 14 bis 17).

Schließlich können die Stützelemente 40 wie in dem zweiten Ausführungsbeispiel gezeigt ebenfalls Löcher 42 aufweisen.

Ist die Trennwand 10, 10' sowohl nach dem ersten als auch nach dem zweiten Ausführungsbeispiel an dem Zwischenboden 70 angeordnet, trennt sie den Bereich oberhalb des Zwischenbodens 70 in zwei Teilbereiche. Sind in der Innentür 60 auf Höhe oberhalb des Zwischenbodens 70 zwei kleine Türen 62 nebeneinander angeordnet, ist durch jede der beiden kleinen Türen 62 jeweils ein Teilbereich zugänglich. Die Trennwand 10, 10' kann einfach ohne zusätzliches Werkzeug montiert werden, indem der Einsetzhaken 20 durch die Ausnehmung 74 geführt und das Federelement 30 an der Anschlagkante 75 zum Anliegen gebracht wird. Ein Entfernen der Trennwand 10, 10' ist ebenso ohne zusätzliches Werkzeug auf einfache Art und Weise mögliche, indem das Federelement 30 gegen die Federkraft zusammengedrückt und die Trennwand 10, 10' von dem Zwischenboden 70 abgenommen wird.

### Bezugszeichenliste

- 10: Trennwand
- 10': Trennwand
- 12: Grundkörper
- 12a: Unterkante
- 12b: Oberkante
- 12c: Vorderkante
- 12d: Rückkante
- 12e: Seitenfläche
- 12f: Seitenfläche
- 20: Einsetzhaken
- 20b: Einsetzhaken
- 20b': Einsetzhaken
- 21: Einführöffnung
- 30: Federelement
- 31: Lasche
- 31a: erster Abschnitt
- 31b: zweiter Abschnitt
- 32: Auflageelement
- 33a: erste Knickkante
- 33b: zweite Knickkante
- 33c: dritte Knickkante
- 40: Stützelement
- 42: Loch
- 50: Loch
- 60: Innentür
- 62: kleine Tür
- 70: Zwischenboden
- 72: Grundkörper
- 72a: Vorderkante
- 72b: Rückkante
- 72c: Seitenkante

- 72d: Seitenkante
- 72e: Oberseite
- 72f: Unterseite
- 74: Ausnehmung
- 75: Anschlagkante
- 77: rahmenartiges Element
- 77b: Mittelsteg
- 79: Loch
- 100: Laborschrank
- 110: Gehäuse
- 112: Außentür
- 120: Innenraum
- 121: Bodenwand
- 122: Deckwand
- 123: Rückwand
- 124: Seitenwand
- 125: Seitenwand
- 127: Sicke
- α: Winkel
- β: Winkel
- γ: Winkel
- δ: Winkel

## Patentansprüche

1. Trennwand (10, 10') zum lösbaren Einsetzen in einen Laborschrank (100), beispielsweise einen Inkubator, Kälteschrank, Wärmeschrank, Trockenschrank oder Klimaschrank, wobei die Trennwand (10, 10') einen im Wesentlichen rechteckigen, flachen Grundkörper (12) mit einer Unterkante (12a), einer Oberkante (12b), einer Vorderkante (12c), einer Rückkante (12d) sowie zwei parallelen Seitenflächen (12e, 12f) aufweist, wobei an dem Grundkörper (12) wenigstens ein zur Vorderkante (12c) offener Einsetzhaken (20) und wenigstens ein Federelement (30) zur Erzeugung einer Kraft in Richtung zur Vorderkante (12c) hin angeordnet sind.

2. Trennwand nach Anspruch 1,
**dadurch gekennzeichnet, dass** an dem Grundkörper (12) wenigstens zwei Einsetzhaken (20) angeordnet sind.

3. Trennwand nach einem der vorhergehenden Ansprüche,
**dadurch gekennzeichnet, dass** der Einsetzhaken (20) an der Unterkante (12a) angeordnet ist.

4. Trennwand nach einem der vorhergehenden Ansprüche,
**dadurch gekennzeichnet, dass** das Federelement (10, 10') durch eine abgekantete Lasche (31), insbesondere eine zweifach abgekantete Lasche (31), gebildet ist.

5. Trennwand nach einem der vorhergehenden Ansprüche,
**dadurch gekennzeichnet, dass** das Federelement (30) in etwa V-förmig ausgebildet ist.

6. Trennwand nach einem der vorhergehenden Ansprüche,
**dadurch gekennzeichnet, dass** ein freies Ende des Federelements (30) als Auflageelement (32) ausgebildet ist.

7. Trennwand nach einem der vorhergehenden Ansprüche,
**dadurch gekennzeichnet, dass** an der Unterkante (12a) wenigstens ein Stützelement (40), welches im Wesentlichen senkrecht zu einer Seitenfläche (12e, 12f) ausgebildet ist, angeordnet ist, wobei vorzugsweise wenigstens zwei Stützelemente (40), welche insbesondere in unterschiedliche Richtungen weisen, an der Unterkante (12a) angeordnet sind.

8. Trennwand nach einem der vorhergehenden Ansprüche,
**dadurch gekennzeichnet, dass** das Federelement (30) an der Unterkante (12a), an der Rückkante (12d) in räumlicher Nähe zur Unterkante (12a) oder an dem Stützelement (40) angeordnet ist.

9. Trennwand nach einem der vorhergehenden Ansprüche,
**dadurch gekennzeichnet, dass** der Grundkörper (12) eine Vielzahl von Löchern (50) aufweist und insbesondere als Lochblech ausgebildet ist.

10. Trennwand nach einem der vorhergehenden Ansprüche, **dadurch gekennzeichnet, dass** die Trennwand (10, 10') aus Metall gefertigt ist.

11. Trennwand nach einem der vorhergehenden Ansprüche, **dadurch gekennzeichnet, dass** die Trennwand (10, 10') als Stanz-Biege-Element und insbesondere einteilig ausgebildet ist.

12. Laborschrank (100), beispielsweise Inkubator, Kälteschrank, Wärmeschrank, Trockenschrank oder Klimaschrank, mit einem wenigstens eine Außentür (112) aufweisenden Gehäuse (110), welches wenigstens einen Innenraum (120) aufweist, der durch eine Innentür (60) verschließbar ist, wobei der Innenraum (120) eine Bodenwand (121), eine Deckwand (122), eine Rückwand (123) und zwei Seitenwände (124, 125) aufweist, wobei zwischen den beiden Seitenwänden (124, 125) parallel zur Bodenwand (121) wenigstens ein Zwischenboden (70) angeordnet ist, wobei entweder die Bodenwand (121) und/oder der Zwischenboden (70) eine erste Ausnehmung (74) und eine Anschlagkante (75) aufweisen, und wobei eine Trennwand (10, 10') gemäß einem der vorhergehenden Ansprüche an der Bodenwand (121) und/oder dem Zwischenboden (70) derart angeordnet ist, dass der Einsetzhaken (20) in die Ausnehmung (74) eingreift und das Federelement (30) an der Anschlagkante (75) anliegt.
